# EUROPEAN PATENT SPECIFICATION

(11) **EP 3 726 991 B1**
(45) Date of publication and mention of the grant of the patent: **06.12.2023**
(21) Application number: 18830763.1
(22) Date of filing: 14.12.2018
(51) Int. Cl.: A01N 43/40, A01N 31/02, A01N 25/02, A01P 1/00

(54) **COLOURED DISINFECTANT PREPARATION WITH A CONTENT OF OCTENIDINE DIHYDROCHLORIDE**
GEFÄRBTE DESINFEKTIONSMITTELZUBEREITUNG MIT EINEM GEHALT AN OCTENIDINDIHYDROCHLORID
PRÉPARATION DÉSINFECTANTE COLORÉE À TENEUR EN DICHLORHYDRATE D'OCTÉNIDINE

(30) Priority: 18.12.2017 DE 102017130313
(43) Date of publication of application: 28.10.2020
(73) Proprietor: SCHÜLKE & MAYR GmbH, 22851 Norderstedt (DE)
(72) Inventor: DETTMANN, Andreas, 22840 Norderstedt (DE); BEHRENDS, Sabine, 22851 Norderstedt (DE); LEMBKE, André, 22851 Norderstedt (DE)
(74) Representative: LKGLOBAL Lorenz & Kopf Patentanwalt Attorney at Law PartG mbB
(86) International application number: PCT/EP2018/084914
(87) International publication number: WO 2019/121380

(56) References cited:
- EP-A1- 2 949 345

## Description

The present invention relates to a disinfectant preparation comprising a) octenidine dihydrochloride, b) isopropanol, c) annatto dye and d) water. In addition, the invention relates to a method for preparing the disinfectant preparations. Furthermore, the invention relates to said preparation for use in a method for disinfecting skin. The invention also relates to the use of the preparation for disinfecting skin or for staining skin.

In certain applications of antiseptic disinfectants, a colour marking of the treated surface (such as skin) is desirable, for example prior to a surgical intervention or during an operation. In addition to compositions based on the active ingredient PVP-iodine, which are already coloured due to the colour of the active ingredient (brown-red), there are coloured skin antiseptics on the market, wherein the active ingredients are non-ionic for example (e.g.Kodan^{®} Tincture forte coloured, with 45% by weight 2-propanol, 10% by weight 1-propanol, 0.2% by weight 2-biphenylol, H₂O₂, E104, E110 and E151).

The group of cationic antiseptic active ingredients for skin antiseptics also includes chlorhexidine gluconate ("chlorhexidine" below), which has been put on the US market in formulations with 70% by volume 2-propanol (e.g. the product ChloraPrep^{®} from Carefusion). On account of the high active ingredient concentrations in these products, a potential loss of efficacy on addition of small amounts of dyes does not matter in antiseptics based on chlorhexidine. In principle however, there are concerns about chlorhexidine-containing antiseptics, specifically on account of the potential formation of p-chloroaniline (carcinogen, toxic) as a potential degradation product on storage. In addition, owing to the comparatively low efficacy of chlorhexidine, relatively high active ingredient concentrations in the product are necessary.

WO2007/130981 A2 describes aqueous solutions of chlorhexidine and a cationic dye. EP 2 499 913 A1 is also concerned with antiseptic solutions containing chlorhexidine, and anionic dyes are proposed for the staining thereof.

WO2016/059653 A1 discloses blue-coloured chlorhexidine solutions for disinfecting skin.

WO97/46622 A1 describes the use of natural or nature-identical synthetic dyes for marking or staining materials. By way of example, the brief marking of operation areas on the skin by means of disinfectant solutions containing dye or colour pens is mentioned. The fact that certain active ingredients of disinfectants have a remanence effect, which must not be influenced by the dye used, is not addressed in WO97/46622 A1.

DE 40 33 690 A1 discloses adducts of norbixin with water-soluble or water-dispersible proteins or branched-chain or cyclic polysaccharides. Norbixin is a carotenoid having two carboxylic acid groups obtained from annatto seeds. Bixin is the monomethyl ester of norbixin. The adducts are used for colouring milk for example. The staining of skin by coloured disinfectants is not addressed.

The medicament and surgical skin antiseptic octeniderm^{®} is approved as a colourless product (Schülke & Mayr GmbH, Norderstedt, Federal Republic of Germany). It contains 0.1% by weight octenidine dihydrochloride ("octenidine" for short below) as remanent active ingredient (and 30% by weight 1-propanol, 45% by weight 2-propanol, remainder: demineralized water). Octenidine is a quaternary ammonium compound of the imine type (cation) with chloride as anion and its antiseptic effectiveness may be inhibited by the vast majority of anionic dyes. Since octeniderm^{®} contains only 0.1% octenidine, dyes added at relatively high concentration (up to 0.5%, such as in products containing chlorhexidine) for staining the skin may lead to a long-term (up to 24 h) distinct decrease in the remanent effect of octenidine.

It has been found that the triphenylmethane dye Patent Blue V, despite its anionic character, exhibits no restriction of the efficacy of octenidine in octeniderm^{®}. However, blue dyes are not accepted on the market on account of the poor visibility of vessels under the skin.

DE 10 2014 107 412 A1 discloses non-solid disinfectant preparations which comprise a) one or more bispyridinium alkanes and b) one or more dyes selected from xanthene dyes, azo dyes and polyterpene compounds, wherein the preparation is free of fluorescein and salts thereof. When an aliphatic alcohol is used alone, the use of n-propanol is preferred over the use of isopropanol. An example preparation comprises 30% by weight n-propanol and 45% by weight isopropanol in addition to octenidine dihydrochloride and annatto dye.

EP 2949345 A1 relates to a non-solid disinfectant preparation which comprises a) bispyridiniumalkane (in particular octenidine) and b) dye selected from xanthene dyes, azo dyes and polyterpene compounds. The preparation is free from fluorescein and salts thereof.

It is an object of the present invention to provide coloured disinfectant preparations in which the efficacy of octenidine is not inhibited and which are eligible for approval even with the added dye. It is also an object of the present invention to provide preparations of this kind which may be stored over a prolonged period and in so doing do not have a tendency to form precipitates that are disadvantageous when dosing, specifically without having to add additional auxiliaries (which merely prevent the formation of precipitates or the inhibition of the antiseptic effect and which contribute nothing to the antiseptic effect). In addition, it is an object of the invention to provide disinfectant preparations which, on application to the skin, do not adversely modify its natural pH. Furthermore, it is an object of the invention to further increase the stability of the dye.

It has now been proven that this object is achieved by a disinfectant preparation obtainable by
(i) dissolving
   c) annatto dye, wherein the amount of annatto dye is 0.005 to 0.05% by weight, specified as norbixin and based on the weight of the disinfectant preparation,
   d) in water,
   wherein the pH is 10.5 to 12.5;
(ii) adding b) at least 50% by weight isopropanol, and
(iii) adding a) 0.02 to 0.25% by weight octenidine dihydrochloride.

The invention is based, inter alia, on preparations which have a particularly good storage stability in the defined pH range. They are outstandingly effective due to the high isopropanol content, including a remanent effect due to the octenidine content. It was surprising, furthermore, that the preparation according to the invention is colour-stable, although a corresponding preparation formulated with chlorhexidine is not colour-stable. It was also surprising that the disinfectant preparations according to the invention can be formulated without adding an additional buffer system, which is why the natural pH of the skin is not adversely altered on application.

### Preparations according to the invention

### a) Octenidine dihydrochloride

Component a), octenidine dihydrochloride, is introduced in preparations according to the invention in an amount of 0.02 to 0.25% by weight, based on the weight of the preparation. Said amount of octenidine dihydrochloride is preferably 0.03 to 0.225% by weight, more preferably 0.04 to 0.215% by weight, especially 0.05 to 0.2% by weight, such as about 0.05% by weight, or about 0.1% by weight, or about 0.2% by weight. Said amount of octenidine dihydrochloride is particularly preferably 0.08 to 0.12% by weight, more preferably 0.09 to 0.11% by weight, based in each case on the weight of the preparation.

### b) Isopropanol

The preparation according to the invention comprises at least 50% by weight isopropanol, preferably 53 to 80% by weight isopropanol, more preferably 55 to 75% by weight isopropanol, in particular 58 to 70% by weight isopropanol, more preferably 60 to 65% by weight isopropanol, such as about 63% by weight isopropanol.

### c) Annatto dye

The preparation comprises annatto dye as component c), wherein the amount of annatto dye is 0.005 to 0.05% by weight, specified as norbixin and based on the weight of the disinfectant preparation. The preparation preferably comprises 0.01 to 0.04% by weight annatto dye, in particular 0.015 to 0.025% by weight annatto dye, more preferably 0.017 to 0.022% by weight annatto dye, such as about 0.02% by weight annatto dye, specified in each case as norbixin and based on the weight of the disinfectant preparation.

Norbixin (a xanthophyll) is a natural dye which is obtained by extracting annatto from the seeds of urucum or achiote plants (Bixa orellana). Norbixin may be obtained by removing the outer layer of the prepared seeds of the annatto shrub (Bixa orellana) by grinding the seeds in cold water and subsequent extraction with solvents such as acetone, methanol or hexane for example. The solution resulting therefrom is acidified bixin (specifically the methyl ester of the acid norbixin), which is then filtered, dried and the precipitate milled. The precipitate mainly comprises cis- and trans-bixin, wherein the principle dye is cis-bixin. Norbixin can be obtained as the corresponding salt using alkaline sodium hydroxide solution, wherein cis-norbixin is again the principle dye.

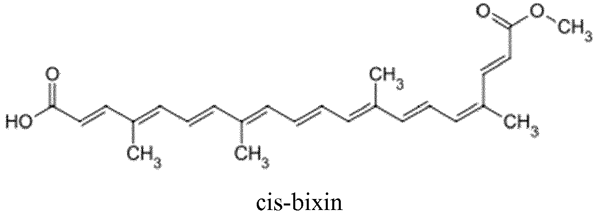

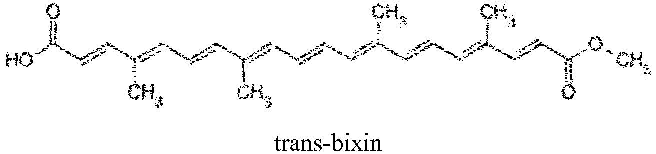

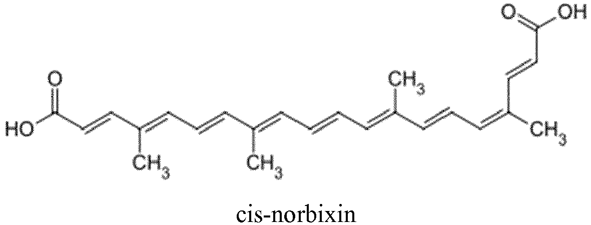

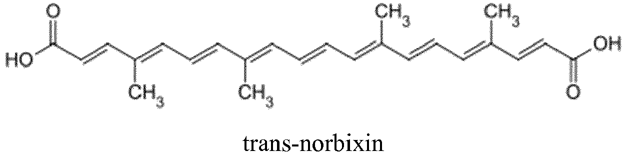

In preparations according to the invention, preferably neither fluorescein nor salts thereof (such as the disodium salt, uranine) are present.

### d) Water

Preparations according to the invention also contain d) water.

Preparations according to the invention may be formulated without a mandatory buffer system and as a result do not unnecessarily influence the natural pH of (human) skin. Preferred preparations according to the invention do not comprise any added buffer system.

Preferred preparations according to the invention comprise less than 35% by weight n-propanol, preferably less than 25% by weight n-propanol, more preferably less than 15% by weight n-propanol, especially less than 5% by weight n-propanol, wherein the preparation particularly preferably does not comprise any n-propanol.

Preferred preparations according to the invention comprise less than 30% by weight ethanol, preferably less than 20% by weight ethanol, more preferably less than 10% by weight ethanol, especially less than 5% by weight ethanol, wherein the preparation particularly preferably does not comprise any ethanol.

Preferred preparations according to the invention comprise less than 10% by weight aromatic alcohol, preferably less than 5% by weight aromatic alcohol, wherein the preparation particularly preferably does not comprise any aromatic alcohol.

Preferred preparations according to the invention do not comprise any glycerol, wherein the preparation preferably does not comprise any polyol.

Preferred preparations according to the invention do not comprise any chloride, acetate or gluconate salt of 1,1'-hexamethylenebis[5-(4-chlorophenyl)biguanide], preferably do not comprise any biguanide or salt thereof at all.

An example formulation of preparations according to the invention is specified below (figures in % by weight):

| | Preferably/more preferably/especially about |
|---|---|
| Octenidine | 0.03 to 0.225/0.04 to 0.215/0.05 to 0.2 |
| 2-Propanol | 53 to 80/55 to 75/58 to 70 |
| Annatto dye | 0.01 to 0.04/0.015 to 0.025/0.017 to 0.022⁺ |

| | |
|---|---|
| ⁺ specified in each case as norbixin and based on the weight of the disinfectant preparation | |

Preferred preparations according to the invention are obtainable using
a) 0.03 to 0.225% by weight octenidine dihydrochloride, preferably about 0.04 to 0.215% by weight octenidine dihydrochloride,
b) 53 to 80% by weight isopropanol, preferably 55 to 75% by weight isopropanol, especially 58 to 70% by weight isopropanol, such as about 63% by weight isopropanol,
c) annatto dye, wherein the amount of annatto dye is 0.01 to 0.04% by weight, specified as norbixin and based on the weight of the disinfectant preparation (especially 0.015 to 0.025, such as about 0.02% by weight, specified as norbixin and based on the weight of the disinfectant preparation), and
d) water,
wherein the pH of the disinfectant preparation is about 11 to 12, measured in the disinfectant preparation without components a) and b).

### Dosage forms of the preparation

The disinfectant preparation according to the invention is a liquid aqueous-alcoholic preparation. The preparation according to the invention is preferably transparent. This facilitates the observation of disinfected areas.

### Method for preparing the preparation according to the invention

The preparation according to the invention cannot be prepared by dissolving octenidine in an aqueous phase, independently of whether the aqueous phase has already been adjusted to the desired pH or already comprises the dye. Instead, it is necessary to formulate octenidine in the aqueous-alcoholic phase. It is also not possible to formulate the annatto dye in an already formulated aqueous-alcoholic phase. In accordance with the invention, therefore, the procedure is such that the dye is dissolved in the aqueous solution at the desired pH, in the range of 10.5 to 12.5, isopropanol is then added and finally the active ingredient octenidine is added. It is thus possible in accordance with the invention, surprisingly, to prepare the preparations specified which - optionally after filtration following addition of the octenidine active ingredient - are storage-stable even over a prolonged period.

The invention therefore also relates to a method in which
i. an aqueous alkaline solution is initially charged,
ii. the annatto dye is added,
iii. the pH of the solution is adjusted to the desired value in the range of 10.5 to 12.5,
iv. isopropanol is added and then
v. octenidine dihydrochloride is added.

### Application

The disinfectant preparations according to the invention are used on the skin in a customary manner, particularly on human skin. In accordance with the present invention, the preparation is preferably used for skin antisepsis of undamaged skin. Preparations according to the invention are preferably applied to the skin in customary manner using swabs.

In this aspect, the invention also relates to a disinfectant preparation for non-medical use in a method for disinfecting skin, especially in humans, wherein the preparation is obtainable by
(i) dissolving
   c) annatto dye, wherein the amount of annatto dye is 0.005 to 0.05% by weight, specified as norbixin and based on the weight of the disinfectant preparation,
   d) in water,
   wherein the pH is 10.5 to 12.5;
(ii) adding b) at least 50% by weight isopropanol, and
(iii) adding a) 0.03 to 0.25% by weight octenidine dihydrochloride, such as about 0.1% by weight octenidine dihydrochloride.

The preparation is preferably employed for non-medical use in a method in which the method is a i) pre-operative measure, ii) a measure prior to puncture or iii) a measure prior to injection. By way of example, punctures or injections are (cf. hygiene requirements in punctures and injections - recommendation of the commission on hospital hygiene and prevention of infection at the Robert Koch Institute (RKI), Federal Law Gazette 2011, 54:1135-1144, DOI 10.1007/s00103-011-1352-8, Springer-Verlag 2011, Tab. I therein :
- s.c. injection,
- s.c. injection with catheter system for s.c. infusion,
- i.m. injection,
- diagnostic lumbar puncture,
- lumbar puncture with single injection,
- peridural/intraspinal anaesthesia,
- joint puncture,
- intravitreal injection,
- kidney biopsy,
- amniocentesis chorionic villus biopsy,
- percutaneous endoscopic gastrostomy (PEG) and
- external ventricular drainage.

The invention also relates to the non-medical use of the preparation for disinfecting (preferably human) skin.

The invention also relates to the non-medical use of the preparation for staining (preferably human) skin.

The advantages of the invention are particularly apparent from the following examples. Amounts refer to weight unless otherwise stated.

### Examples

Materials used:
- (purified) water
- aqueous potassium hydroxide solution (10%, 45%)
- montebixin 401 (pulverulent product having an 80% norbixin content, produced by Frutarom Peru S.A., Lima, Peru)
- hydrochloric acid (10%)
- isopropanol
- octenidine dihydrochloride (octenidine)
- chlorhexidine digluconate (CHX), used as aqueous solution (20% (m/V))

### Example 1: Preparations with octenidine dihydrochloride

To prepare preparations 1A to 1F, purified water was initially charged in each case and then montebixin and potassium hydroxide solution were added and dissolved with stirring (ca. 2h).

The pH was then adjusted to the desired value with potassium hydroxide solution or hydrochloric acid ("xxx" = was used). Isopropanol was then added and dissolved with stirring and finally octenidine was added and dissolved with stirring to give preparations 1A to 1F (Table 1). Only preparations 1D and 1E are illustrative of the invention.

**Table 1. Constituents of preparations 1A to 1F**

| **No.** | **Constituent, parts by weight** | **1A** | **1B** | **1C** | **1D** | **1E** | **1F** |
|---|---|---|---|---|---|---|---|
| **1** | Water | 36.5 75 | 36.5 75 | 36.5 75 | 36.5 75 | 36.5 75 | 36.3 15 |
| **2** | KOH 10% | 0.20 0 | 0.20 0 | 0.20 0 | 0.20 0 | 0.20 0 | --- |
| | KOH 45% | --- | --- | --- | --- | --- | 0.46 0 |
| **3** | Montebixin 401 | 0.02 5 | 0.02 5 | 0.02 5 | 0.02 5 | 0.02 5 | 0.02 5 |
| **4** | KOH 10% | --- | --- | --- | --- | xxx | --- |
| | KOH 45% | --- | --- | --- | --- | --- | xxx |
| **5** | Hydrochloric acid 10% | xxx | xxx | xxx | xxx | --- | --- |
| **6** | Isopropanol | 63.1 00 | 63.1 00 | 63.1 00 | 63.1 00 | 63.1 00 | 63.1 00 |
| **7** | Octenidine | 0.10 0 | 0.10 0 | 0.10 0 | 0.10 0 | 0.10 0 | 0.10 0 |
| | Total | 100. 000 | 100. 000 | 100. 000 | 100. 000 | 100. 000 | 100. 000 |
| | pH (after addition No. 4/5): | 8.0 | 9.0 | 10.0 | 11.0 | 12.0 | 13.0 |

The results of the investigations of the stability of preparations 1A to 1F with octenidine are reported below in Tables 3 to 8 (n.c. = not conducted. S = solution P = precipitate). These results show that preparations with an octenidine and annatto dye content are only sufficiently storage-stable in the narrow pH range of 10.5 to 12.5.

These stability results after 6 months therefore show a higher annatto stability when the pH in the aqueous phase (water + annatto) is adjusted to a narrow highly alkaline pH range. These formulations were then admixed with the required amount of alcohol, applied to the skin and the skin pH measured before and after application. The skin pH was modified and influenced relatively little.

### Example 2: Preparations with 2.0% chlorhexidine digluconate (m/v) (reference example)

To prepare preparations 2A to 2F, the procedure as in Example 1 was followed, with the difference that the active ingredient chlorhexidine gluconate was added in the final step instead of octenidine, to give preparations 2A to 2F, the composition of which is reported in Table 2.

**Table 2. Constituents of preparations 2A to (not according to the invention)**

| **N o.** | **Constituent, parts by weight** | **2A** | **2B** | **2C** | **2D** | **2E** | **2F** |
|---|---|---|---|---|---|---|---|
| **1** | Water | 24.5 7 | 24.5 7 | 24.5 7 | 24.5 7 | 24.5 7 | 24.3 1 |
| **2** | KOH 10% | 0.20 | 0.20 | 0.20 | 0.20 | 0.20 | --- |
| | KOH 45% | --- | --- | --- | --- | --- | 0.46 |
| **3** | Montebixin 401 | 0.02 5 | 0.02 5 | 0.02 5 | 0.02 5 | 0.02 5 | 0.02 5 |
| **4** | KOH 10% | --- | --- | --- | --- | xxx | --- |
| | KOH 45% | --- | --- | --- | --- | --- | xxx |
| **5** | Hydrochloric acid 10% | xxx | xxx | xxx | xxx | --- | --- |
| **6** | Isopropanol | 63.1 00 | 63.1 00 | 63.1 00 | 63.1 00 | 63.1 00 | 63.1 00 |
| **7** | CHX 20% (m/V) | 12.1 05 | 12.1 05 | 12.1 05 | 12.1 05 | 12.1 05 | 12.1 05 |
| | Total | 100. 000 | 100. 000 | 100. 000 | 100. 000 | 100. 000 | 100. 000 |
| | pH (after addition No. 4/5): | 8.0 | 9.0 | 10.0 | 11.0 | 12.0 | 13.0 |

The results of the investigations of the stability of preparations 2A to 2F with a chlorhexidine gluconate content are reported below in Tables 9 to 14 (n.c. = not conducted. S = solution P = precipitate). It follows that preparations with a chlorhexidine gluconate and annatto dye content are not storage-stable, even in the pH range of 10.5 to 12.5, whereas this pH has proven to be particularly advantageous for preparations according to the invention with an octenidine and annatto dye content.

**Table 3. Stability of formulation 1A (pH 8.0)**

| **Test parameter** | | **Method** | **Blank value** | **3 months at** | | **6 months at** | |
|---|---|---|---|---|---|---|---|
| | | | | **-5°C** | **25°C** | **-5°C** | **25°C** |
| Appearance of the solution | | organoleptic | clear, orange-red | clear, vibrant orange + fine flakes in S. | clear, orange | clear, vibrant orange + fine flakes in S. | clear, orange |
| Isopropanol (%) | | GC | 63.2 | n.c. | n.c. | 62.6 | 63.1 |
| Octenidine (%) | | HPLC | 0.100 | n.c. | n.c. | 0.101 | 0.101 |
| Dye absorption maximum | | UV/Vis spectrometry | 449 | | | | |
| Dye extinction ^{§} | | | 1.10 | 0.72 | 0.43 | 0.53 | 0.33 |
| Dye recovery (%) (based on blank value) | | | --- | 65 | 39 | 48 | 30 |
| L*a*b* - values | L* | M0041-01 | 78.3 | 79.0 | 81.5 | 81.6 | 83.0 |
| | a* | | 27.5 | 25.2 | 21.9 | 21.6 | 19.3 |
| | b* | | 130.5 | 131.2 | 134.6 | 134.2 | 136.0 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 4. Stability of formulation 1B (pH 9.0)**

| **Test parameter** | | **Method** | **Blank value** | **3 months at** | | **6 months at** | |
|---|---|---|---|---|---|---|---|
| | | | | **-5°C** | **25°C** | **-5°C** | **25°C** |
| Appearance of the solution | | organoleptic | clear, orange-red | clear, vibrant orange + fine flakes in S. | clear, orange | clear, vibrant orange + fine flakes in S. | clear, orange |
| Isopropanol (%) | | GC | 63.2 | n.c. | n.c. | 63.1 | 62.3 |
| Octenidine (%) | | HPLC | 0.101 | n.c. | n.c. | 0.102 | 0.103 |
| Dye absorption maximum ^{§} | | UV/Vis spectrometry | 449 | | | | |
| Dye extinction (calculated on the basis of 500 mg/25 ml) | | | 1.00 | 0.87 | 0.42 | 0.79 | 0.31 |
| Dye recovery (%) (based on blank value) | | | --- | 87 | 42 | 79 | 31 |
| L*a*b* - values | L* | M0041-01 | 79.5 | 78.0 | 81.7 | 79.8 | 83.5 |
| | a* | | 26.2 | 27.1 | 21.7 | 24.9 | 18.3 |
| | b* | | 132.1 | 129.8 | 134.7 | 132.1 | 136.4 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 5. Stability of formulation 1C (pH 10.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | clear, vibrant orange + fine flakes in S. | clear, orange | clear, vibrant orange + fine flakes in S. | clear, orange |
| Isopropanol (%) | | GC | 63.2 | n.c. | n.c. | 62.6 | 62.8 |
| Octenidine (%) | | HPLC | 0.101 | n.c. | n.c. | 0.102 | 0.102 |
| Dye absorption maximum | | UV/Vis spectrometry | 448 | | | | |
| Dye extinction ^{§} | | | 1.17 | 0.73 | 0.38 | 0.51 | 0.26 |
| Dye recovery (%) (based on blank value) | | | --- | 62 | 32 | 44 | 22 |
| L*a*b* - values | L* | M0041-01 | 78.6 | 79.7 | 83.2 | 82.9 | 85.3 |
| | a* | | 27.0 | 23.7 | 17.2 | 17.6 | 13.4 |
| | b* | | 130.8 | 131.9 | 135.4 | 134.7 | 137.1 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 6. Stability of formulation 1D (pH 11.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | clear, orange-red | clear, orange-red | clear, orange-red | clear, orange-red |
| Isopropanol (%) | | GC | 63.4 | n.c. | n.c. | 62.5 | 62.9 |
| Octenidine (%) | | HPLC | 0.100 | n.c. | n.c. | 0.102 | 0.101 |
| Dye absorption maximum | | UV/Vis spectrometry | 448 | | | | |
| Dye extinction ^{§} | | | 1.40 | 1.40 | 1.33 | 1.18 | 1.28 |
| Dye recovery (%) (based on blank value) | | | --- | 100 | 95 | 84 | 91 |
| L*a*b* - values | L* | M0041-01 | 76.5 | 76.0 | 77.3 | 77.6 | 78.0 |
| | a* | | 30.4 | 30.7 | 28.7 | 27.9 | 27.7 |
| | b* | | 127.9 | 127.1 | 128.7 | 129.1 | 129.6 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 7. Stability of formulation 1E (pH 12.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | clear, orange-red | clear, orange-red | clear, orange-red | clear, orange-red |
| Isopropanol (%) | | GC | 63.2 | n.c. | n.c. | 63.1 | 63.5 |
| Octenidine (%) | | HPLC | 0.100 | n.c. | n.c. | 0.101 | 0.100 |
| Dye absorption maximum | | UV/Vis spectrometry | 447 | | | | |
| Dye extinction ^{§} | | | 1.59 | 1.56 | 1.49 | 1.64 | 1.47 |
| Dye recovery (%) (based on blank value) | | | --- | 98 | 94 | 103 | 92 |
| L*a*b* - values | L* | M0041-01 | 76.0 | 75.8 | 77.0 | 76.2 | 77.9 |
| | a* | | 30.9 | 31.2 | 29.2 | 30.5 | 27.9 |
| | b* | | 127.2 | 126.9 | 128.6 | 127.2 | 129.4 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 8. Stability of formulation 1F (pH 13.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | clear, orange-red | clear, orange-red | clear, orange-red | clear, orange-red |
| Isopropanol (%) | | GC | 63.5 | n.c. | n.c. | 62.2 | 62.4 |
| Octenidine (%) | | HPLC | 0.098 | n.c. | n.c. | 0.095 ^{#} | 0.087 ^{#} |
| Dye absorption maximum | | UV/Vis spectrometry | 447 | | | | |
| Dye extinction ^{§} | | | 1.52 | 1.52 | 1.41 | 1.50 | 1.37 |
| Dye recovery (%) (based on blank value) | | | --- | 100 | 93 | 99 | 90 |
| L*a*b* - values | L* | M0041-01 | 77.0 | 76.6 | 78.1 | 77.3 | 79.2 |
| | a* | | 29.1 | 29.6 | 26.8 | 28.8 | 25.3 |
| | b* | | 128.6 | 127.9 | 129.8 | 128.7 | 131.1 |

| | | | | | | | |
|---|---|---|---|---|---|---|---|
| ^{#} = a peak occurs underneath the internal standard, the samples were therefore calculated using external calibration ^{§} = 500 mg of the formulation were diluted to 25 ml with 96% ethanol by volume and subsequently measured at the absorption maximum. | | | | | | | |

**Table 9. Stability of formulation 2A (pH 8.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | Clear, vibrant orange | Clear, vibrant orange | Clear, vibrant orange | clear, orange |
| Isopropanol (%) | | GC | 63.2 | n.c. | n.c. | 62.8 | 63.2 |
| CHX (%) | | HPLC | 2.33 | n.c. | n.c. | 2.30 | 2.27 |
| Dye absorption maximum | | UV/Vis spectrometry | 450 | | | | |
| Dye extinction ^{§} | | | 1.05 | 0.46 | 0.33 | 0.31 | 0.22 |
| Dye recovery (%) (based on blank value) | | | --- | 44 | 31 | 30 | 21 |
| | L* | | 78.5 | 80.0 | 83.0 | 84.2 | 86.1 |
| L*a*b* - values | a* | M0041-01 | 28.2 | 22.2 | 17.3 | 14.7 | 10.4 |
| | b* | | 131.1 | 132.4 | 135.3 | 136.2 | 136.4 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 10. Stability of formulation 2B (pH 9.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | Clear, vibrant orange | Clear, vibrant orange | Clear, vibrant orange | clear, orange |
| Isopropanol (%) | | GC | 63.3 | n.c. | n.c. | 62.8 | 62.2 |
| CHX (%) | | HPLC | 2.33 | n.c. | n.c. | 2.29 | 2.28 |
| Dye absorption maximum | | UV/Vis spectrometry | 450 | | | | |
| Dye extinction ^{§} | | | 1.12 | 0.55 | 0.38 | 0.34 | 0.25 |
| Dye recovery (%) (based on blank value) | | | --- | 49 | 34 | 30 | 22 |
| L*a*b* - values | L* | M0041-01 | 78.4 | 80.1 | 82.9 | 84.1 | 86.0 |
| | a* | | 28.1 | 21.8 | 17.5 | 14.8 | 10.7 |
| | b* | | 130.9 | 132.1 | 135.4 | 136.0 | 136.5 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 11. Stability of formulation 2C (pH 10.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | Clear, vibrant orange | Clear, vibrant orange | Clear, vibrant orange | clear, orange |
| Isopropanol (%) | | GC | 63.1 | n.c. | n.c. | 62.9 | 62.7 |
| CHX (%) | | HPLC | 2.32 | n.c. | n.c. | 2.31 | 2.28 |
| Dye absorption maximum | | UV/Vis spectrometry | 450 | | | | |
| Dye extinction ^{§} | | | 1.16 | 0.49 | 0.39 | 0.34 | 0.26 |
| Dye recovery (%) (based on blank value) | | | --- | 42 | 34 | 29 | 22 |
| L*a*b* - values | L* | M0041-01 | 78.2 | 80.1 | 82.9 | 84.2 | 85.9 |
| | a* | | 28.4 | 21.0 | 17.7 | 14.0 | 10.9 |
| | b* | | 130.7 | 132.1 | 135.4 | 135.7 | 136.4 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 12. Stability of formulation 2D (pH 11.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | Clear, vibrant orange (somewhat darker) | Clear, vibrant orange | Clear, vibrant orange | Clear, vibrant orange |
| Isopropanol (%) | | GC | 63.2 | n.c. | n.c. | 62.2 | 63.0 |
| CHX (%) | | HPLC | 2.34 | n.c. | n.c. | 2.32 | 2.28 |
| Dye absorption maximum | | UV/Vis spectrometry | 450 | | | | |
| Dye extinction ^{§} | | | 1.29 | 0.85 | 0.54 | 0.34 | 0.26 |
| Dye recovery (%) (based on blank value) | | | --- | 66 | 42 | 26 | 20 |
| L*a*b* - values | L* | M0041-01 | 77.1 | 77.3 | 82.2 | 82.1 | 84.9 |
| | a* | | 30.6 | 27.4 | 19.9 | 19.8 | 13.5 |
| | b* | | 129.2 | 129.0 | 135.0 | 134.6 | 136.4 |

| | | | | | | | |
|---|---|---|---|---|---|---|---|
| ^{§} = 500 mg of the formulation were diluted to 25 ml with 96% ethanol by volume and subsequently measured at the absorption maximum. | | | | | | | |

**Table 13. Stability of formulation 2E (pH 12.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | clear, red-orange | clear, orange-red | clear, red-orange | clear, orange-red |
| Isopropanol (%) | | GC | 63.5 | n.c. | n.c. | 63.0 | 63.0 |
| CHX (%) | | HPLC | 2.34 | n.c. | n.c. | 2.30 | 2.24 ^{#} |
| Dye absorption maximum | | UV/Vis spectrometry | 449 | | | | |
| Dye extinction ^{§} | | | 1.42 | 1.31 | 1.12 | 0.56 | 0.36 |
| Dye recovery (%) (based on blank value) | | | --- | 92 | 79 | 39 | 25 |
| L*a*b* - values | L* | M0041-01 | 75.5 | 73.6 | 77.4 | 76.0 | 79.6 |
| | a* | | 33.0 | 31.8 | 29.2 | 30.8 | 25.6 |
| | b* | | 126.9 | 123.5 | 129.4 | 127.3 | 132.0 |

| | | | | | | | |
|---|---|---|---|---|---|---|---|
| ^{#} = additional peaks in the chromatogram => degradation products ^{§} = 500 mg of the formulation were diluted to 25 ml with 96% ethanol by volume and subsequently measured at the absorption maximum. | | | | | | | |

**Table 14. Stability of formulation 2F (pH 13.0)**

| **Test parameter** | | **Method** | **Blank value** | **3 months** | | **6 months** | |
|---|---|---|---|---|---|---|---|
| | | | | **-5°C** | **25°C** | **-5°C** | **25°C** |
| Appearance of the solution | | organoleptic | clear, orange-red | cloudy, red-orange + fine P. and needle-like crystals | clear, orange-red + crystals | cloudy, red-orange + fine P. and needle-like crystals | clear, orange-red + crystals |
| Isopropanol (%) | | GC | 63.4 | n.c. | n.c. | 62.9 | 63.1 |
| CHX (%) | | HPLC | 1.77 | n.c. | n.c. | 1.26 ^{#} | 1.04 ^{#} |
| Dye absorption maximum | | UV/Vis spectrometry | 448 | | | | |
| Dye extinction ^{§} | | | 1.45 | 1.40 | 1.25 | 1.34 | 1.15 |
| Dye recovery (%) (based on blank value) | | | --- | 97 | 86 | 92 | 79 |
| L*a*b* - values | L* | M0041-01 | 76.4 | 68.9 | 77.4 | 75.7 | 78.9 |
| | a* | | 30.4 | 28.7 | 27.4 | 27.9 | 24.1 |
| | b* | | 128.0 | 115.0 | 129.0 | 126.3 | 130.6 |

| | | | | | | | |
|---|---|---|---|---|---|---|---|
| ^{#} = additional peaks in the chromatogram => degradation products ^{§} = 500 mg of the formulation were diluted to 25 ml with 96% ethanol by volume and subsequently measured at the absorption maximum. | | | | | | | |

## Claims

1. Disinfectant preparation obtainable by
(i) dissolving
c) annatto dye, wherein the amount of annatto dye is 0.005 to 0.05% by weight, specified as norbixin and based on the weight of the disinfectant preparation,
d) in water,
wherein the pH is 10.5 to 12.5;
(ii) adding b) at least 50% by weight isopropanol, and
(iii) adding a) 0.02 to 0.25% by weight octenidine dihydrochloride.

2. Preparation according to Claim 1, **characterized in that** said preparation is obtainable by adding 0.03 to 0.225% by weight octenidine dihydrochloride, preferably 0.04 to 0.215% by weight octenidine dihydrochloride.

3. Preparation according to Claim 1 or 2, **characterized in that** said preparation comprises 53 to 80% by weight isopropanol, preferably 55 to 75% by weight isopropanol, more preferably 58 to 70% by weight isopropanol, especially 60 to 65% by weight isopropanol, such as about 63% by weight isopropanol.

4. Preparation according to any of the preceding claims, **characterized in that** said preparation comprises 0.01 to 0.04% by weight annatto dye, specified as norbixin and based on the weight of the disinfectant preparation, preferably 0.015 to 0.025% by weight annatto dye, especially about 0.02% by weight annatto dye.

5. Preparation according to any of the preceding claims, **characterized in that** said preparation comprises no additional buffer system.

6. Preparation according to any of the preceding claims, **characterized in that** said preparation comprises less than 35% by weight n-propanol, preferably less than 25% by weight n-propanol, more preferably less than 15% by weight n-propanol, especially less than 5% by weight n-propanol, wherein the preparation particularly preferably does not comprise any n-propanol.

7. Preparation according to any of the preceding claims, **characterized in that** said preparation comprises less than 30% by weight ethanol, preferably less than 20% by weight ethanol, more preferably less than 10% by weight ethanol, especially less than 5% by weight ethanol, wherein the preparation particularly preferably does not comprise any ethanol.

8. Preparation according to any of the preceding claims, **characterized in that** said preparation comprises less than 10% by weight aromatic alcohol, preferably less than 5% by weight aromatic alcohol, wherein the preparation particularly preferably does not comprise any aromatic alcohol.

9. Preparation according to any of the preceding claims, **characterized in that** said preparation does not comprise any glycerol, wherein the preparation preferably does not comprise any polyol.

10. Preparation according to any of the preceding claims, **characterized in that** said preparation does not comprise any chloride, acetate or gluconate salt of 1,1'-hexameth-ylenebis[5-(4-chlorophenyl)biguanide], preferably does not comprise any biguanide or salt thereof.

11. Preparation according to any of the preceding claims, **characterized in that** said preparation is obtainable using
a) 0.03 to 0.225% by weight octenidine dihydrochloride, preferably about 0.04 to 0.215% by weight octenidine dihydrochloride,
b) 53 to 80% by weight isopropanol, preferably 55 to 75% by weight isopropanol, especially 58 to 70% by weight isopropanol, such as about 63% by weight isopropanol,
c) annatto dye, wherein the amount of annatto dye is 0.01 to 0.04% by weight, specified as norbixin and based on the weight of the disinfectant preparation (especially 0.015 to 0.025, such as about 0.02% by weight, specified as norbixin and based on the weight of the disinfectant preparation), and
d) water,
wherein the pH of the disinfectant preparation is about 11 to 12, measured in the disinfectant preparation without components a) and b).

12. Method for preparing the preparation according to any of the preceding claims, in which
i. an aqueous alkaline solution is initially charged,
ii. the annatto dye is added,
iii.the pH of the solution is adjusted to the desired value in the range of 10.5 to 12.5,
iv. isopropanol is added and then
v. octenidine dihydrochloride is added.

13. Non-medical use of a disinfectant preparation in a method for disinfecting skin, especially human skin, wherein the preparation is obtainable by
(i) dissolving
c) annatto dye, wherein the amount of annatto dye is 0.005 to 0.05% by weight, specified as norbixin and based on the weight of the disinfectant preparation,
d) in water,
wherein the pH is 10.5 to 12.5;
(ii) adding b) at least 50% by weight isopropanol, and
(iii) adding a) 0.03 to 0.25% by weight octenidine dihydrochloride, such as about 0.1% by weight octenidine dihydrochloride.

14. Non-medical use according to Claim 13, wherein the method is a i) pre-operative measure, ii) a measure prior to puncture or iii) a measure prior to injection.

15. Non-medical use of a preparation according to any of Claims 1 to 11 in disinfecting skin or in staining skin.

## Patentansprüche

1. Desinfizierende Zubereitung, die erhältlich ist durch
(i) Lösen von
c) Annatto-Farbstoff, wobei die Menge an Annatto-Farbstoff 0,005 bis 0,05 Gew.-% beträgt, angegeben als Norbixin und bezogen auf das Gewicht der desinfizierenden Zubereitung,
d) in Wasser,
wobei der pH-Wert 10,5 bis 12,5 beträgt;
(ii) Zugeben b) von mindestens 50 Gew.-% Isopropanol, und
(iii) Zugeben c) von 0,02 bis 0,25 Gew.-% Octenidindihydrochlorid.

2. Zubereitung nach Anspruch 1, **dadurch gekennzeichnet, dass** die Zubereitung erhältlich ist durch Zugeben von 0,03 bis 0,225 Gew.-% Octenidindihydrochlorid, vorzugsweise 0,04 bis 0,215 Gew.-% Octenidindihydrochlorid.

3. Zubereitung nach Anspruch 1 oder 2, **dadurch gekennzeichnet, dass** die Zubereitung 53 bis 80 Gew.-% Isopropanol umfasst, vorzugsweise 55 bis 75 Gew.-% Isopropanol, bevorzugter 58 bis 70 Gew.-% Isopropanol, insbesondere 60 bis 65 Gew.-% Isopropanol, wie etwa 63 Gew.-% Isopropanol.

4. Zubereitung nach einem der vorhergehenden Ansprüche, **dadurch gekennzeichnet, dass** die Zubereitung 0,01 bis 0,04 Gew.-% Annatto-Farbstoff umfasst, angegeben als Norbixin und bezogen auf das Gewicht der desinfizierenden Zubereitung, vorzugsweise 0,015 bis 0,025 Gew.-% Annatto-Farbstoff, insbesondere etwa 0,02 Gew.-% Annatto-Farbstoff.

5. Zubereitung nach einem der vorhergehenden Ansprüche, **dadurch gekennzeichnet, dass** die Zubereitung kein zusätzliches Puffersystem umfasst.

6. Zubereitung nach einem der vorhergehenden Ansprüche, **dadurch gekennzeichnet, dass** die Zubereitung weniger als 35 Gew.-% n-Propanol umfasst, vorzugsweise weniger als 25 Gew.-% n-Propanol, bevorzugter weniger als 15 Gew.-% n-Propanol, insbesondere weniger als 5 Gew.-% n-Propanol, wobei die Zubereitung besonders bevorzugt kein n-Propanol umfasst.

7. Zubereitung nach einem der vorhergehenden Ansprüche, **dadurch gekennzeichnet, dass** die Zubereitung weniger als 30 Gew.-% Ethanol umfasst, vorzugsweise weniger als 20 Gew.-% Ethanol, bevorzugter weniger als 10 Gew.-% Ethanol, insbesondere weniger als 5 Gew.-% Ethanol, wobei die Zubereitung besonders bevorzugt kein Ethanol umfasst.

8. Zubereitung nach einem der vorhergehenden Ansprüche, **dadurch gekennzeichnet, dass** die Zubereitung weniger als 10 Gew.-% aromatischen Alkohol umfasst, vorzugsweise weniger als 5 Gew.-% aromatischen Alkohol, wobei die Zubereitung besonders bevorzugt keinen aromatischen Alkohol umfasst.

9. Zubereitung nach einem der vorhergehenden Ansprüche, **dadurch gekennzeichnet, dass** die Zubereitung kein Glycerin umfasst, wobei die Zubereitung vorzugsweise kein Polyol umfasst.

10. Zubereitung nach einem der vorhergehenden Ansprüche, **dadurch gekennzeichnet, dass** die Zubereitung kein Chlorid-, Acetat- oder Gluconat-Salz von 1,1'-Hexamethylenbis[5-(4-chlorphenyl)-biguanid] umfasst, vorzugsweise kein Biguanid oder Salz davon umfasst.

11. Zubereitung nach einem der vorhergehenden Ansprüche, **dadurch gekennzeichnet, dass** die Zubereitung erhältlich ist unter Verwendung von
a) 0,03 bis 0,225 Gew.-% Octenidindihydrochlorid, vorzugsweise etwa 0,04 bis 0,215 Gew.-% Octenidindihydrochlorid,
b) 53 bis 80 Gew.-% Isopropanol, vorzugsweise 55 bis 75 Gew.-% Isopropanol, insbesondere 58 bis 70 Gew.-% Isopropanol, wie etwa 63 Gew.-% Isopropanol,
c) Annatto-Farbstoff, wobei die Menge an Annatto-Farbstoff 0,01 bis 0,04 Gew.-% beträgt, angegeben als Norbixin und bezogen auf das Gewicht der desinfizierenden Zubereitung (insbesondere 0,015 bis 0,025, wie etwa 0,02 Gew.-%, angegeben als Norbixin und bezogen auf das Gewicht der desinfizierenden Zubereitung), und
d) Wasser,
wobei der pH-Wert der desinfizierenden Zubereitung etwa 11 bis 12 beträgt, gemessen in der desinfizierenden Zubereitung ohne die Komponenten a) und b).

12. Verfahren zur Herstellung der Zubereitung nach einem der vorhergehenden Ansprüche, bei dem
i. eine wässrige alkalische Lösung vorgelegt wird,
ii. der Annatto-Farbstoff zugegeben wird,
iii. der pH-Wert der Lösung auf den gewünschten Wert im Bereich von 10,5 bis 12,5 eingestellt wird,
iv. Isopropanol zugegeben wird und dann
v. Octenidindihydrochlorid zugegeben wird.

13. Nicht-medizinische Verwendung einer desinfizierenden Zubereitung in einem Verfahren zur Desinfektion von Haut, insbesondere menschlicher Haut, wobei die Zubereitung erhältlich ist durch
(i) Lösen von
c) Annatto-Farbstoff, wobei die Menge an Annatto-Farbstoff 0,005 bis 0,05 Gew.-% beträgt, angegeben als Norbixin und bezogen auf das Gewicht der desinfizierenden Zubereitung,
d) in Wasser,
wobei der pH-Wert 10,5 bis 12,5 beträgt;
(ii) Zugeben b) von mindestens 50 Gew.-% Isopropanol, und
(iii) Zugeben c) von 0,02 bis 0,25 Gew.-% Octenidindihydrochlorid, wie etwa 0,1 Gew.-% Octenidindihydrochlorid.

14. Nicht-medizinische Verwendung nach Anspruch 13, wobei das Verfahren eine i) präoperative Maßnahme, ii) eine Maßnahme vor Punktion oder iii) eine Maßnahme vor Injektion ist.

15. Nicht-medizinische Verwendung einer Zubereitung nach einem der Ansprüche 1 bis 11 zur Desinfektion von Haut oder zum Einfärben von Haut.

## Revendications

1. Préparation désinfectante pouvant être obtenue par
(i) dissolution
c) d'un colorant d'annatto, dans laquelle la quantité de colorant d'annatto est de 0,005 à 0,05 % en poids, spécifié comme la norbixine et sur la base du poids de la préparation désinfectante,
d) dans l'eau,
dans laquelle le pH est de 10,5 à 12,5 ;
(ii) ajout b) d'au moins 50 % en poids d'isopropanol, et
(iii) ajout a) de 0,02 à 0,25 % en poids de dichlorhydrate d'octénidine.

2. Préparation selon la revendication 1, **caractérisée en ce que** ladite préparation peut être obtenue par ajout de 0,03 à 0,225 % en poids de dichlorhydrate d'octénidine, de préférence de 0,04 à 0,215 % en poids de dichlorhydrate d'octénidine.

3. Préparation selon la revendication 1 ou 2, **caractérisée en ce que** ladite préparation comprend de 53 à 80 % en poids d'isopropanol, de préférence de 55 à 75 % en poids d'isopropanol, de manière d'avantage préférée de 58 à 70 % en poids d'isopropanol, notamment de 60 à 65 % en poids d'isopropanol, par exemple environ 63 % en poids d'isopropanol.

4. Préparation selon l'une quelconque des revendications précédentes, **caractérisée en ce que** ladite préparation comprend de 0,01 à 0,04 % en poids de colorant d'annatto, spécifié comme la norbixine et sur la base du poids de la préparation désinfectante, de préférence de 0,015 à 0,025 % en poids de colorant d'annatto, notamment environ 0,02 % en poids de colorant d'annatto.

5. Préparation selon l'une quelconque des revendications précédentes, **caractérisée en ce que** ladite préparation ne comprend pas de système tampon supplémentaire.

6. Préparation selon l'une quelconque des revendications précédentes, **caractérisée en ce que** ladite préparation comprend moins de 35 % en poids de n-propanol, de préférence moins de 25 % en poids de n-propanol, de manière davantage préférée moins de 15 % en poids de n-propanol, notamment moins de 5 % en poids de n-propanol, dans laquelle de manière particulièrement préférée la préparation ne comprend pas du tout de n-propanol.

7. Préparation selon l'une quelconque des revendications précédentes, **caractérisée en ce que** ladite préparation comprend moins de 30 % en poids d'éthanol, de préférence moins de 20 % en poids d'éthanol, de manière davantage préférée moins de 10 % en poids d'éthanol, notamment moins de 5 % en poids d'éthanol, dans laquelle de manière particulièrement préférée la préparation ne comprend pas du tout d'éthanol.

8. Préparation selon l'une quelconque des revendications précédentes, **caractérisée en ce que** ladite préparation comprend moins de 10 % en poids d'alcool aromatique, de préférence moins de 5 % en poids d'alcool aromatique, dans laquelle de manière particulièrement préférée la préparation ne comprend pas du tout d'alcool aromatique.

9. Préparation selon l'une quelconque des revendications précédentes, **caractérisée en ce que** ladite préparation ne comprend pas du tout de glycérol, dans laquelle la préparation ne comprend de préférence pas du tout de polyol.

10. Préparation selon l'une quelconque des revendications précédentes, **caractérisée en ce que** ladite préparation ne comprend pas du tout de sel de chlorure, d'acétate ou de glutamate de 1,1'-hexaméthylènebis[5-(4-chlorophényl)biguanide], de préférence ne comprend pas du tout de biguanide ou de sel de celui-ci.

11. Préparation selon l'une quelconque des revendications précédentes, **caractérisée en ce que** ladite préparation peut être obtenue en utilisant
a) de 0,03 à 0,225 % en poids de dichlorhydrate d'octénidine, de préférence de 0,04 à 0,215 % en poids de dichlorhydrate d'octénidine,
b) de 53 à 80 % en poids d'isopropanol, de préférence de 55 à 75 % en poids d'isopropanol, notamment de 58 à 70 % en poids d'isopropanol, par exemple environ 63 % en poids d'isopropanol,
c) un colorant d'annatto, dans laquelle la quantité de colorant d'annatto est de 0,01 à 0,04 % en poids, spécifié comme la norbixine et sur la base du poids de la préparation désinfectante (notamment de 0,015 à 0,025, par exemple environ 0,02 % en poids, spécifié comme la norbixine et sur la base du poids de la préparation désinfectante), et
d) de l'eau,
dans laquelle le pH de la préparation désinfectante est d'environ 11 à 12, mesuré dans la préparation désinfectante sans les composants a) et b).

12. Procédé de préparation de la préparation selon l'une quelconque des revendications précédentes, dans lequel
i. une solution alcaline aqueuse est initialement chargée,
ii. le colorant d'annatto est ajouté,
iii. le pH de la solution est ajusté à la valeur souhaitée dans la plage de 10,5 à 12,5,
iv. de l'isopropanol est ajouté puis
v. du dichlorhydrate d'octénidine est ajouté.

13. Utilisation non médicale d'une préparation désinfectante dans un procédé de désinfection de la peau, notamment de la peau humaine, dans lequel la préparation peut être obtenue par
(i) dissolution
c) de colorant d'annatto, dans lequel la quantité de colorant d'annatto est de 0,005 à 0,05 % en poids, spécifié comme la norbixine et sur la base du poids de la préparation désinfectante,
d) dans l'eau,
dans lequel le pH est de 10,5 à 12,5 ;
(ii) ajout b) d'au moins 50 % en poids d'isopropanol, et
(iii) ajout a) de 0,03 à 0,25 % en poids de dichlorhydrate d'octénidine, par exemple d'environ 0,1 % en poids de dichlorhydrate d'octénidine.

14. Utilisation non médicale selon la revendication 13, dans laquelle le procédé est i) une mesure préopératoire, ii) une mesure avant une ponction ou iii) une mesure avant une injection.

15. Utilisation non médicale d'une préparation selon l'une quelconque des revendications 1 à 11 dans la désinfection de la peau ou la coloration de la peau.
